(19) 

Europäisches
Patentamt
European
Patent Office
Office européen
des brevets

(11) **EP 4 681 731 A1**

(12) **EUROPEAN PATENT APPLICATION**
published in accordance with Art. 153(4) EPC

(43) Date of publication:
**21.01.2026 Bulletin 2026/04**

(21) Application number: **24774081.4**

(22) Date of filing: **18.03.2024**

(51) International Patent Classification (IPC):
**A61K 39/395** $^{(2006.01)}$    **C07K 16/28** $^{(2006.01)}$
**C12N 15/13** $^{(2006.01)}$    **A61K 47/68** $^{(2017.01)}$

(52) Cooperative Patent Classification (CPC):
**A61K 39/395; A61K 47/68; A61P 17/00;**
**A61P 17/04; A61P 29/00; A61P 37/02;**
**C07K 16/00; C07K 16/28**

(86) International application number:
**PCT/CN2024/082273**

(87) International publication number:
**WO 2024/193516 (26.09.2024 Gazette 2024/39)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **17.03.2023 CN 202310270735**
**28.09.2023 CN 202311282257**

(71) Applicant: **Bio-Thera Solutions, Ltd.**
**Guangzhou, Guangdong 510530 (CN)**

(72) Inventors:
 • **LIANG, Shizhong**
  **Guangzhou, Guangdong 510530 (CN)**
 • **LIU, Xiong**
  **Guangzhou, Guangdong 510530 (CN)**

 • **LI, Huijie**
  **Guangzhou, Guangdong 510530 (CN)**
 • **WANG, Hao**
  **Guangzhou, Guangdong 510530 (CN)**
 • **SONG, Shuqiang**
  **Guangzhou, Guangdong 510530 (CN)**
 • **YU, Jin-Chen**
  **Guangzhou, Guangdong 510530 (CN)**
 • **LI, Shengfeng**
  **Guangzhou, Guangdong 510530 (CN)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference
Table(s) and the Sequence Listing(s) can be
downloaded from the EPO website

(54) **USE OF ANTI-OX40 ANTIBODY IN TREATMENT OF INFLAMMATIONS OR IMMUNE DISEASES**

(57) A use of an anti-OX40 antibody in treatment of inflammatory or immune diseases, and a treatment method for treating inflammatory or immune diseases. The treatment method comprises administering an effective amount of an anti-OX40 antibody to a patient in need.

FIG. 6

**Description**

**TECHNICAL FIELD**

The present disclosure pertains to the field of bio-pharmaceuticals, and particularly relates to use of an anti-OX40 antibody in treating an inflammatory or immune disease.

**BACKGROUND**

OX40, also known as TNFRSF4, ACT35, CD134, etc., belongs to the tumor necrosis factor receptor superfamily (TNFRSF), and is also a type I transmembrane glycoprotein. In mice and humans, OX40 is primarily expressed on activated T cells, including CD4+ T cells, CD8+ T cells, Th1, Th2, and Th17 cells, as well as CD4+ Foxp3 regulatory T cells (Tregs). OX40 is also expressed at a lower level on activated Tregs, neutrophils, natural killer (NK) cells, and natural killer T (NKT) cells. The activation of T cell subsets such as Th2 by OX40 (CD134) may play a role in the pathology of inflammatory skin diseases such as AD.

At present, it is widely believed that the anti-OX40 antibody activates the immune system and inhibits tumors mainly through the following three cell physiological mechanisms. The first one is to directly activate CD4$^+$ and CD8$^+$ effector T cells, thereby promoting their proliferation, survival, and secretion of related inflammatory factors; the second one is to inhibit the signaling and activity of Tregs, thereby reducing their inhibitory effects on the immune system; the third one is to deplete Tregs through mechanisms such as antibody-dependent cell-mediated cytotoxicity (ADCC) or antibody dependent cellular phagocytosis (ADCP), thereby diminishing their inhibition on effector T cells. In animal models of autoimmune diseases, blocking OX40 signaling can inhibit T cell responses and pathogenic symptoms.

Atopic dermatitis (AD) is a chronic, recurrent, and inflammatory skin disease with diverse clinical manifestations. Its most fundamental features include dry skin, chronic eczematous skin lesions, and significant itching. Immune abnormality, skin barrier dysfunction, skin flora disturbance, and the like are key factors in the pathogenesis of AD. Lesional and even seemingly normal skin in atopic dermatitis is often accompanied by skin flora disturbance, characterized by increased colonization of *Staphylococcus aureus* and reduced flora diversity, as well as resulting functional abnormalities such as metabolic disruptions, which contribute to the progression of skin inflammation.

**SUMMARY**

In one aspect, the present disclosure discloses a method or use of an anti-OX40 antibody or an antigen-binding fragment for treating an inflammatory or immune disease.

In some embodiments, the method or use of the anti-OX40 antibody or the antigen-binding fragment for treating an inflammatory or immune disease comprises administering to a patient in need thereof an effective amount of the anti-OX40 antibody or the antigen-binding fragment.

In some embodiments, the anti-OX40 antibody or the antigen-binding fragment comprises at least one or more of HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

In some embodiments, the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

In some embodiments, a heavy chain variable region of the anti-OX40 antibody or the antigen-binding fragment comprises a sequence set forth in SEQ ID NO: 7, or a sequence having at least 80% identity to the sequence set forth in SEQ ID NO: 7, or an amino acid sequence having one or more conservative amino acid substitutions as compared to the sequence set forth in SEQ ID NO: 7; and/or

a light chain variable region of the anti-OX40 antibody or the antigen-binding fragment comprises a sequence set forth in SEQ ID NO: 8, or a sequence having at least 80% identity to the sequence set forth in SEQ ID NO: 8, or an amino acid sequence having one or more conservative amino acid substitutions as compared to the sequence set forth in SEQ ID NO: 8.

In some embodiments, the heavy chain variable region of the anti-OX40 antibody or the antigen-binding fragment comprises a sequence set forth in SEQ ID NO: 7, and the light chain variable region of the anti-OX40 antibody or the antigen-binding fragment comprises a sequence set forth in SEQ ID NO: 8.

In some embodiments, a heavy chain of the anti-OX40 antibody comprises a sequence set forth in SEQ ID NO: 9, or a sequence having at least 80% identity to the sequence set forth in SEQ ID NO: 9, or an amino acid sequence having one or more conservative amino acid substitutions as compared to the sequence set forth in SEQ ID NO: 9; and/or

a light chain of the anti-OX40 antibody comprises a sequence set forth in SEQ ID NO: 10, or a sequence having at least 80% identity to the sequence set forth in SEQ ID NO: 10, or an amino acid sequence having one or more conservative amino acid substitutions as compared to the sequence set forth in SEQ ID NO: 10.

In some embodiments, the anti-OX40 antibody is antibody M or antibody M-KF, a heavy chain of antibody M or antibody M-KF comprises a sequence set forth in SEQ ID NO: 9, and a light chain of antibody M or antibody M-KF comprises a sequence set forth in SEQ ID NO: 10; antibody M or antibody M-KF comprises two heavy chains having the same sequence and two light chains having the same sequence.

Table 1. Amino acid sequences of antibody M and antibody M-KF (classified according to Kabat)

| Name | SEQ ID NO | Amino acid sequence |
|---|---|---|
| HCDR1 | 1 | SYGMH |
| HCDR2 | 2 | VIWEDGSNQYYADSVKG |
| HCDR3 | 3 | DNQDTSPDVGIDY |
| LCDR1 | 4 | RASQNISPFLN |
| LCDR2 | 5 | AAVGLQS |
| LCDR3 | 6 | QQYTDYPLT |
| VH | 7 | QVQLVESGGGVVQPGESLRLSCAASGFTFSSYGMHWVRQAPGK GLEWVAVIWEDGSNQYYADSVKGRFTISRDNSKNTLYLQMNSLR AEDTAVYYCAKDNQDTSPDVGIDYWGQGTLVTVSS |
| VL | 8 | DIQMTQSPSSLSASVGDRVTITCRASQNISPFLNWYQQKPGKAPK LLIYAAVGLQSGVPSRFSGSGSGTDFTLTISSLQPEDFATYYCQQYT DYPLTFGGGTKVEIK |
| Heavy chain | 9 | QVQLVESGGGVVQPGESLRLSCAASGFTFSSYGMHWVRQAPGK GLEWVAVIWEDGSNQYYADSVKGRFTISRDNSKNTLYLQMNSLR AEDTAVYYCAKDNQDTSPDVGIDYWGQGTLVTVSSASTKGPSVF PLAPSSKSTSGGTAALGCLVKDYFPEPVTVSWNSGALTSGVHTFP AVLQSSGLYSLSSVVTVPSSSLGTQTYICNVNHKPSNTKVDKKVE PKSCDKTHTCPPCPAPELLGGPSVFLFPPKPKDTLMISRTPEVTCV VVDVSHEDPEVKFNWYVDGVEVHNAKTKPREEQYNSTYRVVSV LTVLHQDWLNGKEYKCKVSNKALPAPIEKTISKAKGQPREPQVY TLPPSRDELTKNQVSLTCLVKGFYPSDIAVEWESNGQPENNYKTT PPVLDSDGSFFLYSKLTVDKSRWQQGNVFSCSVMHEALHNHYTQ KSLSLSPGK |
| Light chain | 10 | DIQMTQSPSSLSASVGDRVTITCRASQNISPFLNWYQQKPGKAPK LLIYAAVGLQSGVPSRFSGSGSGTDFTLTISSLQPEDFATYYCQQYT DYPLTFGGGTKVEIKRTVAAPSVFIFPPSDEQLKSGTASVVCLLNN FYPREAKVQWKVDNALQSGNSQESVTEQDSKDSTYSLSSTLTLS KADYEKHKVYACEVTHQGLSSPVTKSFNRGEC |

In some embodiments, the anti-OX40 antibody (e.g., antibody M-KF) or the antigen-binding fragment has a fucosylation level of 0-10%. In some embodiments, the anti-OX40 antibody (e.g., antibody M-KF) or the antigen-binding fragment has a fucosylation level of 0-5%. In some embodiments, the anti-OX40 antibody (e.g., antibody M-KF) or the antigen-binding fragment has a fucosylation level of about 0%, about 0.1%, about 0.5%, about 0.8%, about 1%, about 1.3%, about 1.6%, about 2.1%, 2.9%, about 3%, about 3.3%, about 3.8%, about 4%, about 4.2%, 4.3%, about 4.6%, about 5%, or a range between any two of these values (inclusive) or any value therein. In some embodiments, the anti-OX40 antibody (e.g., antibody M-KF) or the antigen-binding fragment does not bind to fucose.

In some embodiments, the anti-OX40 antibody (e.g., antibody M-KF) or the antigen-binding fragment has an enhanced ADCC effect, and the antibody of the present disclosure selectively depletes OX40+ activated T cells by the ADCC effect.

In some embodiments, the anti-OX40 antibody or the antigen-binding fragment can be expressed in CHO cells or HEK293 cells by genetic engineering and obtained by purification; the purification can be performed by conventional methods, such as performing centrifugation on the cell suspension and collecting the supernatant, and then performing centrifugation again to further remove impurities. Protein A affinity column, ion exchange column, and other methods may be used for purifying the antibody protein.

In some embodiments, the anti-OX40 antibody or the antigen-binding fragment is expressed by a cell line in which an α-(1,6)-fucosyltransferase gene is knocked out, such as the CHO-BAT-KF cell line disclosed in ZL201810910890.8. In some embodiments, antibody M-KF is expressed by a CHO-BAT-KF cell line.

The anti-OX40 antibody or the antigen-binding fragment used in the present disclosure is disclosed in WO2021190431.

In some embodiments, the inflammatory or immune disease includes, but is not limited to, graft-versus-host disease, inflammatory diseases, or autoimmune diseases.

In some embodiments, the inflammatory or immune disease is an OX40-associated inflammatory or autoimmune disease.

In some embodiments, the present disclosure discloses a method or use of an anti-OX40 antibody or an antigen-binding fragment for treating graft-versus-host disease, which comprises administering to a patient in need thereof an effective amount of the anti-OX40 antibody or the antigen-binding fragment. In some embodiments, the method or use is to treat, relieve, or prevent the onset frequency, duration, or severity of one or more adverse symptoms or reactions (e.g., weight loss, hair loss, rash, hematuria, ascites, and inflammatory cell infiltration or death in the liver, intestine, or lung) associated with graft-versus-host disease, thereby inducing alleviation or regression of graft-versus-host disease, or preventing graft-versus-host disease. In some embodiments, the graft may include bone marrow, hematopoietic stem cells, peripheral blood stem cells, or umbilical cord blood stem cells.

In some embodiments, the present disclosure discloses a method or use of an anti-OX40 antibody or an antigen-binding fragment for treating graft rejection, which comprises administering to a patient in need thereof an effective amount of the anti-OX40 antibody or the antigen-binding fragment. In some embodiments, the method or use is to treat, relieve, or prevent the onset frequency, duration, or severity of one or more adverse symptoms or reactions (e.g., weight loss, hair loss, rash, hematuria, ascites, and inflammatory cell infiltration or death in the liver, intestine, or lung) associated with graft rejection (e.g., immune response or tissue destruction against the graft or grafted cells), thereby inducing alleviation or regression of graft rejection, or preventing graft rejection. In some embodiments, the graft may include cells, tissues, or organs of the kidney, heart, lung, skin, liver, or pancreas.

In some embodiments, the present disclosure discloses a method or use of an anti-OX40 antibody or an antigen-binding fragment for treating, relieving, or preventing graft-versus-host disease (GVHD) symptoms in a graft-versus-host disease model, and alleviating or regressing graft-versus-host disease in a graft-versus-host disease model (e.g., immunodeficient (NCG) mice).

In some embodiments, the present disclosure discloses a method or use of an anti-OX40 antibody or an antigen-binding fragment for treating, relieving, or preventing an inflammatory disease, which comprises administering to a patient in need thereof an effective amount of the anti-OX40 antibody or the antigen-binding fragment. In some embodiments, the inflammation is present in the lung, joint, muscle, skin, central or peripheral nervous system, or intestine. In some embodiments, the inflammation is present in the skin. In some embodiments, the disease is a chronic inflammatory skin disease. In some embodiments, the anti-OX40 antibody or the antigen-binding fragment is used to treat, relieve, or prevent the onset frequency, duration, or severity of one or more adverse symptoms or reactions associated with the inflammation. In some embodiments, the anti-OX40 antibody or the antigen-binding fragment may also be used to treat, relieve, block, or prevent an inflammation caused by an individual's response to an infectious agent (e.g., a bacterial, viral, or parasitic infectious agent).

In some embodiments, the present disclosure discloses a method or use of an anti-OX40 antibody or an antigen-binding fragment for treating, relieving, or preventing an autoimmune disease, which comprises administering to a patient in need thereof an effective amount of the anti-OX40 antibody or the antigen-binding fragment. In some embodiments, the autoimmune disease is selected from rheumatoid arthritis, multiple sclerosis, diabetes, Crohn's disease, inflammatory bowel disease, ulcerative colitis, celiac disease, psoriasis, proliferative lupus nephritis, granulomatous myopathy, and polymyositis.

In some embodiments, the OX40-associated inflammatory or autoimmune disease is atopic dermatitis, asthma, inflammatory bowel disease, graft rejection, autoimmune diabetes, graft-versus-host disease (GVHD), or experimental autoimmune encephalomyelitis (EAE).

In some embodiments, the OX40-associated inflammatory or autoimmune disease is atopic dermatitis. In some embodiments, the OX40-associated inflammatory or autoimmune disease is moderate-to-severe atopic dermatitis. In some embodiments, the OX40-associated inflammatory or autoimmune disease is moderate-to-severe atopic dermatitis that cannot be adequately controlled by previous topical medications or that is not amenable to treatment with topical medications.

In some embodiments, the present disclosure discloses a method for treating an inflammatory or immune disease, which comprises administering an effective amount of an anti-OX40 antibody or an antigen-binding fragment, wherein the anti-OX40 antibody or the antigen-binding fragment at a dose of about 0.6 mg to 1500 mg is administered each time.

In some embodiments, the anti-OX40 antibody can be formulated into a pharmaceutical composition and administered to a patient in a variety of forms suitable for the chosen route of administration, such as parenteral, intravenous (iv), intramuscular, topical, or subcutaneous (sc) route. In some embodiments, the anti-OX40 antibody can be intravenously infused. The dose of the anti-OX40 antibody will depend on the properties of the drug, the extent of the internalization, transport, and release of the drug triggered at the cell surface, and the disease being treated and the conditions of the patient (e.g., age, sex, body weight, etc.).

In some embodiments, the present disclosure discloses a method for treating an inflammatory or immune disease, which comprises administering to a patient in need thereof an effective amount of an anti-OX40 antibody (or formulation), wherein the anti-OX40 antibody at an effective amount of about 0.6 mg to 1500 mg (or a formulation containing the anti-OX40 antibody at this dose) is administered each time. The dosage schedule and administration route depend on benefit-risk assessment and general clinical practice guidelines for the anti-OX40 antibody (or formulation) in certain patient populations.

In some embodiments, the anti-OX40 antibody (e.g., antibody M or antibody M-KF) at a dose of about 0.06 mg to 1500 mg or a formulation containing the anti-OX40 antibody at this dose is administered each time. In some embodiments, the anti-OX40 antibody (e.g., antibody M or antibody M-KF) at a dose of about 60 mg to 1200 mg or a formulation containing the anti-OX40 antibody at this dose is administered each time. In some embodiments, the anti-OX40 antibody (e.g., antibody M or antibody M-KF) at a dose of about 300 mg to 900 mg or a formulation containing the anti-OX40 antibody at this dose is administered each time. In some embodiments, the anti-OX40 antibody at a dose of about 50 mg, 75 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1200 mg, or 1500 mg, or a range between any two of these values (inclusive) or any value therein, or a formulation containing the anti-OX40 antibody at this dose is administered each time. In some embodiments, the anti-OX40 antibody at a dose of about 300 mg, 600 mg, or 900 mg is administered each time.

In some embodiments, the anti-OX40 antibody (e.g., antibody M or antibody M-KF) at a dose of about 0.01 mg/kg to 25 mg/kg or a formulation containing the anti-OX40 antibody at this dose is administered each time. In some embodiments, the anti-OX40 antibody (e.g., antibody M or antibody M-KF) at a dose of about 1 mg/kg to 20 mg/kg or a formulation containing the anti-OX40 antibody at this dose is administered each time. In some embodiments, the anti-OX40 antibody (e.g., antibody M or antibody M-KF) at a dose of about 5 mg/kg to 15 mg/kg or a formulation containing the anti-OX40 antibody at this dose is administered each time. In some embodiments, the anti-OX40 antibody at a dose of about 0.01 mg/kg, about 0.02 mg/kg, about 0.03 mg/kg, about 0.06 mg/kg, about 0.08 mg/kg, about 0.1 mg/kg, about 0.2 mg/kg, about 0.3 mg/kg, about 0.5 mg/kg, about 0.9 mg/kg, about 1 mg/kg, about 2 mg/kg, about 2.5 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, or a range between any two of these values (inclusive) or any value therein, or a formulation containing the anti-OX40 antibody at this dose is administered each time.

In some embodiments, the anti-OX40 antibody is antibody M-KF or a formulation containing antibody M-KF at this dose. In some embodiments, the anti-OX40 antibody is antibody M or a formulation containing antibody M at this dose.

In some embodiments, the drug is administered about once every 1 week, about once every 2 weeks, about once every 3 weeks, or about once every 4 weeks.

In some embodiments, one treatment cycle is 1 week to 7 weeks. In some embodiments, one treatment cycle is about 1 week, about 2 weeks, about 3 weeks, about 4 weeks, about 5 weeks, about 6 weeks, about 7 weeks, or a range between any two of these values (inclusive) or any value therein. In some embodiments, one treatment cycle is about 1 week, about 2 weeks, about 3 weeks, or about 4 weeks. In some embodiments, one treatment cycle is 2 weeks. In some embodiments, one treatment cycle is 4 weeks. In some embodiments, administration is performed once or twice per cycle. In some embodiments, the anti-OX40 antibody (or formulation) at the dose described above is additionally administered in week 2 after the administration in cycle 1.

In some embodiments, the anti-OX40 antibody (or formulation) is administered to the patient once per treatment cycle. In some embodiments, the anti-OX40 antibody (or formulation) is administered multiple times, e.g., 2, 3, 4, or 5 times, per treatment cycle. In some embodiments, administration is performed twice in treatment cycle 1.

In some embodiments, the patient is treated for one treatment cycle. In some embodiments, the patient is treated for multiple (e.g., 2, 3, 4, 5, 6, or 7) treatment cycles. In some embodiments, the patient is treated until the condition is alleviated and no treatment is required.

In some embodiments, after a single administration, the patient's symptoms are alleviated. In some embodiments, after a single administration, the patient's symptoms have not been alleviated as expected, and then about 0.6 mg to 1500 mg of the anti-OX40 antibody is administered to the patient until the patient's symptoms are alleviated.

In some embodiments, the present disclosure provides a method or use of an anti-OX40 antibody (or formulation) in combination with an additional treatment method for treating, alleviating, or preventing an inflammatory or immune disease. In some embodiments, the present disclosure provides a method or use of an anti-OX40 antibody (or formulation) in combination with an additional therapeutic agent for treating an inflammatory or immune disease.

In one aspect, provided is a method for inhibiting or preventing an OX40-mediated cellular response, which comprises administering to an individual in need of inhibition or prevention of an OX40-mediated cellular response an effective amount of an antibody (or formulation). In some embodiments, provided is a method for modulating OX40-mediated cell signaling, which comprises administering to an individual in need of modulation of OX40-mediated cell signaling an effective amount of an antibody (or formulation). In some embodiments, the methods of the present disclosure comprise using an anti-OX40 antibody to induce depletion of OX40-expressing T cells via antibody-dependent cell-mediated cytotoxicity (ADCC).

In some embodiments, the methods of the present disclosure can regulate, alleviate, attenuate, inhibit, block, or treat an OX40-associated inflammatory or immune disease by blocking the binding of OX40 to OX40L or by depleting OX40+ T cells via ADCC.

In one aspect, the present disclosure provides a method for inhibiting lymphocyte proliferation by using an anti-OX40 antibody or an antigen-binding fragment. In one aspect, the present disclosure provides a method for reducing the number of activated T cells in the blood, spleen, lymph node, intestine, liver, lung, or skin of a graft-versus-host disease (GVHD) model by using an anti-OX40 antibody or an antigen-binding fragment. In one aspect, the present disclosure provides a method for reducing inflammatory cytokines in the blood, spleen, lymph node, intestine, liver, lung, or skin of a graft-versus-host disease (GVHD) model by using an anti-OX40 antibody or an antigen-binding fragment.

In another aspect, the present disclosure discloses use of an anti-OX40 antibody or an antigen-binding fragment in preparing a medicament for treating an inflammatory or immune disease. In some embodiments, the medicament for treating an OX40-associated inflammatory or immune disease comprises an anti-OX40 antibody. In some embodiments, the anti-OX40 antibody is antibody M. In some embodiments, the anti-OX40 antibody is antibody M-KF. In some embodiments, antibody M-KF is expressed by a cell line in which an $\alpha$-(1,6)-fucosyltransferase gene is knocked out.

In another aspect, the present disclosure further discloses a kit comprising an anti-OX40 antibody (or formulation) and an instruction for directing administration of the anti-OX40 antibody (or formulation) to a patient in need thereof. In some embodiments, the anti-OX40 antibody is antibody M. In some embodiments, the anti-OX40 antibody is antibody M-KF. In some embodiments, antibody M-KF is expressed by a cell line in which an $\alpha$-(1,6)-fucosyltransferase gene is knocked out.

In another aspect, the present disclosure further discloses a pharmaceutical composition, which comprises an anti-OX40 antibody and is suitable for injection, such as a pharmaceutical composition for bolus injection or a pharmaceutical composition for infusion (drip). The pharmaceutical composition suitable for injection includes a sterile aqueous solution (herein water-soluble solution) or dispersion and sterile powders for the instant preparation of a sterile injectable solution or dispersion. For intravenous administration, a suitable carrier includes a solvent or dispersion medium of normal saline, bacteriostatic water or phosphate-buffered saline (PBS), ethanol, or polyol (e.g., glycerol, propylene glycol, and liquid polyethylene glycol), and a suitable mixture thereof. In some embodiments, the pharmaceutical composition further comprises a pharmaceutically acceptable carrier. In some embodiments, the pharmaceutically acceptable carrier may include an antibacterial and/or antifungal agent, e.g., p-hydroxylbenzoate, chlorobutanol, phenol, ascorbic acid and thimerosal. In some embodiments, the pharmaceutically acceptable carrier may include an isotonic agent, such as a sugar, a polyol (such as mannitol and sorbitol), and sodium chloride. In some embodiments, the pharmaceutical composition comprises at least 0.1% anti-OX40 antibody. The percentage of antibody may vary and is between about 2% and 90% by weight of a given dosage form. The amount of anti-OX40 antibody in such a therapeutically useful pharmaceutical composition may be an effective amount for administration. In some embodiments, the anti-OX40 antibody is antibody M. In some embodiments, the anti-OX40 antibody is antibody M-KF. In some embodiments, antibody M-KF is expressed by a cell line in which an $\alpha$-(1,6)-fucosyltransferase gene is knocked out.

In another aspect, the present disclosure further discloses a method for preparing the pharmaceutical composition described above, which comprises mixing the anti-OX40 antibody described herein with a pharmaceutically acceptable carrier suitable for injection (e.g., water for injection and normal saline). The method for mixing the anti-OX40 antibody described above with the pharmaceutically acceptable carrier is generally known in the art.

In another aspect, the present disclosure further discloses use of the pharmaceutical composition described above in treating an inflammatory or immune disease.

## BRIEF DESCRIPTION OF THE DRAWINGS

FIG. 1 shows that antibody M-KF blocks the binding of OX40L to OX40 on the cell surface;

FIG. 2 shows that antibody M-KF blocks OX40L from activating OX40 signals;

FIG. 3 shows the mixed lymphocyte reaction (MLR) of antibodies, where the antibodies from left to right are antibody M-KF, KHK4083, antibody M, hIgG1, and Abatacept;

FIG. 4 shows changes in the body weight of mice in different groups (data are presented as Mean $\pm$ SEM);

FIG. 5 shows GVHD scoring of mice in different groups (data are presented as Mean $\pm$ SEM);

FIG. 6 shows the survival curves of mice in different groups (data are presented as Mean $\pm$ SEM);

FIG. 7 shows the flow cytometry analysis of cell numbers of mice in groups G1-G4 (data are presented as Mean $\pm$ SD);

FIG. 8 shows the flow cytometry analysis of cell numbers of mice in groups G1 and G5-G7 (data are presented as Mean + SD);

FIG. 9 shows an analysis of the CBA detection results of mice in different groups (data are presented as Mean + SEM).

## Terms

Unless otherwise stated, each of the following terms shall have the meaning described below.

### *Definitions*

It should be noted that the term "an" entity refers to one or more of the entities. For example, "an antibody" should be interpreted as one or more antibodies. As such, the terms "a" (or "an"), "one or more" and "at least one" can be used interchangeably herein.

"Conservative amino acid substitution" refers to the substitution of one amino acid residue with another amino acid residue having a side chain (R group) with similar chemical properties (e.g., charge or hydrophobicity). Generally, conservative amino acid substitutions do not substantially alter the functional properties of the protein. Examples of groups of amino acids that have side chains with similar chemical properties include: 1) aliphatic side chains: glycine, alanine, valine, leucine and isoleucine; 2) aliphatic hydroxyl side chains: serine and threonine; 3) amide-containing side chains: asparagine and glutamine; 4) aromatic side chains: phenylalanine, tyrosine and tryptophan; 5) basic side chains: lysine, arginine and histidine; and 6) acidic side chains: aspartic acid and glutamic acid.

The number of amino acids of "conservative amino acid substitutions of VL or VH" may be about 1, about 2, about 3, about 4, about 5, about 6, about 8, about 9, about 10, about 11, about 13, about 14, about 15 conservative amino acid substitutions, or a range between any two of these values (inclusive) or any value therein. The number of amino acids of "conservative amino acid substitutions of a heavy chain or a light chain" may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

"Identity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

"At least 80% identity" refers to about 80% identity, about 81% identity, about 82% identity, about 83% identity, about 85% identity, about 86% identity, about 87% identity, about 88% identity, about 90% identity, about 91% identity, about 92% identity, about 94% identity, about 95% identity, about 98% identity, about 99% identity, or a range between any two of these values (inclusive) or any value therein.

"Antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising, in its molecule, at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (LCDR1-3) and heavy chain CDRs (HCDR1-3).

The term "antibody" includes a wide variety of polypeptides that can be biochemically distinguished. Those skilled in the art will appreciate that the classes of heavy chains include gamma, mu, alpha, delta, or epsilon ($\gamma$, $\mu$, $\alpha$, $\delta$, or $\varepsilon$)

and some subclasses (e.g., γ1-γ4). The nature of this chain determines the "type" of the antibody as IgG, IgM, IgA, IgD, or IgE. Immunoglobulin subclasses (isotypes), such as IgG1, IgG2, IgG3, IgG4, and IgG5, have been well characterized and the functional specificity imparted is also known. All types of immunoglobulins are within the scope of the present disclosure. In some embodiments, the immunoglobulin molecule is an IgG species.

The antibodies, antigen-binding fragments, or derivatives disclosed in the present disclosure include, but are not limited to, polyclonal antibodies, monoclonal antibodies, multispecific antibodies, fully human antibodies, humanized antibodies, primatized antibodies, chimeric antibodies, single-chain variable fragments (scFvs), and epitope-binding fragments (e.g., Fab, Fab', and F(ab')$_2$).

Light chains can be classified into kappa (κ) or lambda (λ). Each heavy chain may bind to a κ or λ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is Vκ; and the immunoglobulin λ light chain variable region is V$_λ$.

Both the light and heavy chains are divided into structurally and functionally homologous regions. The terms "constant" and "variable" are used in accordance with function. The light chain variable region (VL) and heavy chain variable region (VH) determine the antigen recognition and specificity. The light chain constant region (CL) and the heavy chain constant region (CH) impart important biological properties such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

Where the terms used and/or accepted in the art have two or more definitions, the definitions of the terms used herein include all of these meanings unless explicitly indicated as opposite.

One specific example is the use of the term "complementarity determining regions" ("CDRs") to describe non-contiguous antigen-binding sites found within the variable regions of heavy and light chain polypeptides.

CDRs defined according to Kabat and Chothia schemes include overlaps or subsets of amino acid residues when compared with each other. Nevertheless, it is within the scope of the present disclosure to apply any definition to refer to the CDRs of an antibody or a variant thereof. The exact number of residues comprising a specific CDR will vary according to the sequence and size of the CDR. Those skilled in the art can generally determine which specific residues a CDR comprises based on the variable region amino acid sequence of an antibody.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering scheme proposed by Kabat et al., U.S. Dept. of Health and Human Services in Sequence of Proteins of Immunological Interest (1991). EU or Chothia numbering scheme can also be applicable to the antibody.

"About" refers to a conventional error range for corresponding values as readily understood by those skilled in the relevant art. In some embodiments, "about" mentioned herein refers to the values described and ranges of ± 10%, ± 5%, or ± 1% therefrom.

"Effective amount" refers to the amount of an active compound or medicament that results in a biological or medical response in a tissue, system, animal, subject, or human; the effective amount is sought by researchers, vets, doctors or other clinical doctors. An exemplary, non-limiting effective amount (dose) is about 0.1 mg/kg to about 100 mg/kg and any value or range within the range described above. Higher or lower effective amounts (doses) may be administered, such as 0.01-500 mg/kg and any value or range within the range described above. Other exemplary, non-limiting effective amounts (doses) are about 0.5-50 mg/kg, 1.0-25 mg/kg, 1.0-10 mg/kg, and any value or range within the ranges described above.

"Treatment" refers to both therapeutic treatment and prophylactic or preventative measures, the purpose of which is to prevent, slow down, ameliorate, or halt undesirable physiological changes or disorders, such as the progression of disease, including, but not limited to, alleviation of symptoms, diminishment of the extent of disease, stabilization (i.e., not worsening) of disease state, delay or slowing of disease progression, amelioration, palliation, alleviation, or elimination (whether partial or total) of disease state, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the composition disclosed herein for detection, diagnostic procedures, and/or treatment.

"Patient" refers to any mammal in need of diagnosis, prevention, or treatment, including humans, dogs, cats, guinea pigs, rabbits, rats, mice, horses, cattle, and the like.

"In need" means that the patient has been identified as in need of a particular method or treatment. In some embodiments, identification may be made by any diagnostic mode. The patient may be in need of any methods and treatments described herein.

MTD is defined as the highest dose level at which the DLT was explored in ≤1/6 subjects of a certain dose group during the DLT evaluation period.

**Eczema Area and Severity Index (EASI):** EASI is a validated measure for assessing the severity and extent of AD in both clinical practice and trials (Hanifin et al., 2001, Exp. Dermatol. 10:11-18). In the EASI assessment, the severity of 4 clinical features of eczema in 4 body parts (head/neck, trunk, upper limbs, and lower limbs), i.e., erythema (E), induration (edema)/papulation (I), excoriation (Ex), and lichenification (L), is scored from 0 to 3 (0 = none, 1 = mild, 2 = moderate, 3 = severe). Scores of 1.5 and 2.5 are permitted, provided that the severity of any symptom is scored at least 1 (mild). In addition, the eczema area in each of the 4 body regions is scored from 0 to 6 (where 1 palm area of the subject is calculated as 1% of the body surface area) (0 = 0%, 1 = 1% to 9%, 2 = 10% to 29%, 3 = 30% to 49%, 4 = 50% to 69%, 5 = 70% to 89%, 6 = 90% to 100%).

The score for each region is calculated according to the formula: "sum of 4 eczema clinical feature scores $\times$ eczema area score $\times$ region multiplier", where the region multipliers are: 0.1 for head/neck, 0.2 for upper limbs, 0.3 for trunk, and 0.4 for lower limbs. The resulting 4 regional scores are summed to give the total EASI score (maximum 72).

| Body part | EASI score[1-2] |
|---|---|
| Head/neck (H) | (E + I + Ex + L) $\times$ area $\times$ 0.1 |
| Upper limbs (ULs) | (E + I + Ex + L) $\times$ area $\times$ 0.2 |
| Trunk (T) | (E + I+ Ex + L) $\times$ area $\times$ 0.3 |
| Lower limbs (LLs) | (E + I + Ex + L) $\times$ area $\times$ 0.4 |
| EASI = | *Sum of the scores of the 4 body parts described above* |
| E = erythema, I = induration (edema)/papulation, Ex = excoriation, L = lichenification. Note 1: The average severity score for each sign across the 4 body regions is 0-3 (0: none; 1: mild; 2: moderate; 3: severe), with scores of 1.5 and 2.5 being permitted, except for 0.5. Note 2: In the analysis process, the eczema area in each of the 4 body regions is scored from 0 to 6 (where 1 palm area of the subject is calculated as 1% of the body surface area) (0 = 0%, 1 = 1% to 9%, 2 = 10% to 29%, 3 = 30% to 49%, 4 = 50% to 69%, 5 = 70% to 89%, 6 = 90% to 100%). | |

**Investigator's Global Assessment (IGA):** In the IGA, the investigator will assess the subject's overall skin symptoms at each visit using a 5-point scale ranging from 0 (clear) to 4 (severe), to determine the severity of AD and clinical response to treatment. The IGA score is assessed at each clinical visit. The IGA scale is as follows:

| Score | Morphological description |
|---|---|
| 0-clear | No inflammation signs of atopic dermatitis (no erythema, induration (edema)/papulation, lichenification, oozing/crusting). Post-inflammatory pigmentation and/or hypopigmentation may be present. |
| 1-almost clear | Barely perceptible erythema, barely perceptible induration (edema)/papulation, and/or minimal lichenification. No oozing or crusting. |
| 2-mild | Mild but definite erythema (pink), mild but definite induration (edema)/papulation, and/or mild but definite lichenification. No oozing or crusting. |
| 3-moderate | Clearly visible erythema (dark red), clearly visible induration (edema)/papulation, and/or clearly visible lichenification. Oozing and crusting may be present. |
| 4-severe | Marked erythema (deep or bright red), marked induration (edema)/papulation, and/or marked lichenification. Disease is extensive in scope. Oozing or crusting may be present. |

**SCORing Atopic Dermatitis (SCORAD):** In the SCORAD assessment, the skin lesion area of AD is calculated as the sum of percentages for each defined body region, with a maximum score of 100% (designated as "A" in the overall SCORAD calculation). The severity of 6 specific symptoms of AD is assessed using the following scale: 0 = none, 1 = mild, 2 = moderate, 3 = severe, with a maximum score of 18 (designated as "B" in the overall SCORAD calculation). Itch and insomnia are assessed by subjects on a visual analog scale (VAS), where 0 indicates no itch (or insomnia), 10 indicates the most severe itch (or insomnia), with a maximum score of 20 (designated as "C" in the overall SCORAD calculation). The SCORAD score is calculated as A/5 + 7B/2 + C.

**Assessment Form of AD Skin Lesion Body Surface Area (BSA):** Body surface area (BSA) refers to the percentage (%) of the total body surface area affected by AD.

**Itch Severity Numeric Rating Scale (NRS):** The severe itch is assessed by NRS using the visual analog scale (VAS) method. Itching intensity is scored on a scale of 0-10, where 0 indicates "no itch" and 10 indicates "the worst imaginable itch".

**Sleep disturbance Numerical Rating Scale (NRS):** Daily sleep disturbance is assessed by NRS using the visual analog scale (VAS) method. Subjects score their degree of sleep disturbance on a scale of 0-10, ranging from "no sleep loss" (0) to "I could not sleep at all" (10).

**Dermatology Life Quality Index (DLQI):** The DLQI assesses the impact of skin symptoms on daily life over the past week. The DLQI includes 6 subscales (symptoms and feelings, daily activities, leisure, work and school, personal relationships, and treatment), scored based on 10 questions. The DLQI is calculated by summing the scores of each question, yielding a maximum score of 30 and a minimum score of 0. Higher scores indicate more severe impairment of quality of life (QOL).

PK (pharmacokinetic) parameters used herein are defined as follows:

PK parameters following single administration: $C_{max}$, $T_{max}$, $T_{1/2}$, CL, Vd, Ke, MRT, $AUC_{(0-\tau)}$, and $AUC_{(0-\infty)}$.

| | |
|---|---|
| $C_{max}$ | Peak concentration. Directly obtained from the measured plasma concentration-time data. |
| $T_{max}$ | Time to peak. Directly obtained from the measured plasma concentration-time data. |
| $T_{1/2}$ | Terminal elimination half-life. $T_{1/2} = \ln2/\lambda_z$ |
| CL | Apparent clearance. $CL = D_{po}/AUC_{0-\infty}$ |
| Vd | Apparent volume of distribution. $Vd = D_{po}/AUC_{0-\infty}/\lambda_z$ |
| Ke ($\lambda_z$) | Elimination rate constant, a slope of the terminal segment of a semi-logarithmic log of drug concentration-time curve calculated by using the linear regression method. |
| $MRT_{0-t}$ | Mean residence time, calculated by the formula: $AUMC_{0-t}/AUC_{0-t}$, where the calculation formula for $AUMC_{0-t}$ is: $AUMC_{(i, i+1)} = (t_{i+1} - t_i)(t_{i+1} * C_{i+1} - t_i * C_i)/2$, and AUMC is the sum of all $AUMC_{(i, i+1)}$. |
| $MRT_{0-\infty}$ | Calculation formula: $AUMC_{0-\infty}/AUC_{0-\infty}$, where $AUMC_{0-\infty} = AUMC_{0-t} + T_{last} \times \|C_{last}\|/\lambda z \pm \|C_{last}\|/(\lambda z \times \lambda z)$. |
| $AUC_{0-t}$ | Area under the curve from zero to the time at which the lowest plasma concentration can be detected. Calculated by the linear trapezoidal rule: $AUC_{(i, i+1)} = (T_{i+1} - T_i)(C_i + C_{i+1})/2$, where $AUC_{0-t}$ is the sum of all $AUC_{(i, i+1)}$. |
| $AUC_{0-\infty}$ | Area under the curve of time extrapolated from zero to infinity. $AUC_{0-\infty} = AUC_{0-t} + C_t/\lambda_z$ (Ct is the last measurable plasma concentration). |
| $AUC_{\_\%}$ Extrap | The proportion of AUC theoretically extrapolated from the last point to infinity relative to $AUC_{0-\infty}$. |

PK parameters following multiple administrations: $C_{max, ss}$, $C_{avg, ss}$, $C_{min, ss}$, $AUC_{(0-\tau)ss}$, $AUC_{(0-\infty)ss}$, $T_{max, ss}$, $T_{1/2, ss}$, CL, Vss, Ke, MRT, accumulation index (Rac), and fluctuation index DF.

| | |
|---|---|
| $C_{max, ss}$ | Peak concentration at steady state. Directly obtained from the measured plasma concentration-time data at steady state within dosing intervals. |
| $C_{min, ss}$ | Minimum concentration at steady state. Directly obtained from the measured plasma concentration-time data at steady state within dosing intervals. |
| $C_{avg,ss}$ | Mean plasma concentration at steady state within dosing intervals. $C_{av} = AUC_{0-\tau}/\tau$, where $\tau$ is the administration interval. |
| $AUC_{(0-t)ss}$ | Area under the curve at steady state from zero to the time at which the lowest plasma concentration can be detected. Calculated by the linear trapezoidal rule: $AUC_{(i, i+1), ss} = (T_{i+1} - T_i)(C_i + C_{i+1})/2$, where $AUC_{0-t, ss}$ is the sum of all $AUC_{(i, i+1), ss}$. |

(continued)

| | | |
|---|---|---|
| $AUC_{(0-\infty)ss}$ | Area under the curve of time at steady state extrapolated from zero to infinity. $AUC_{0-\infty, ss} = AUC_{0-t, ss} + C_t/\lambda_z$ ($C_t$ is the last measurable plasma concentration at steady state). | |
| $T_{max,ss}$ | Time to peak. Directly obtained from the measured plasma concentration-time data. | |
| $T_{1/2,ss}$ | Terminal elimination half-life. $T_{1/2, ss} = \ln2/\lambda_z$ | |
| $CL_{ss}$ | Apparent clearance at steady state. $CL_{ss} = D_{po}/AUC_{ss}$. | |
| $Vd$ | Volume of distribution at steady state, $Vd = D_{po}/AUC_{0-\infty, ss}/\lambda_z$ | |
| $Ke (\lambda_z)$ | Elimination rate constant, a slope of the terminal segment of a semi-logarithmic log of drug concentration-time curve calculated by using the linear regression method. | |
| $MRT_{0-\infty, ss}$ | Calculation formula: $AUMC_{0-\infty, ss}/AUC_{0-\infty, ss}$, where $AUMC_{0-\infty, ss} = AUMC_{0-t, ss} + T_{last} \times \lvert C_{last}\rvert/\lambda_z \pm \lvert C_{last}\rvert/(\lambda_z \times \lambda_z)$. | |
| Rac | Accumulation ratio. Rac = $\dfrac{1}{1-e^{-Ke\tau}}$ | |
| DF | The degree of fluctuation. $DF = (C_{max, ss} - C_{min, ss})/C_{avg, ss}$ | |
| $AUC_{\_\%\ Extrap}$ | The proportion of AUC theoretically extrapolated from the last point to infinity relative to $AUC_{0-\infty}$. | |

Adverse events (AEs) refer to all adverse medical events occurring in a subject who has been given an experimental drug, which may be manifested as symptoms, signs, diseases, or abnormalities in laboratory tests, but do not necessarily have a causal relationship with the experimental drug.

As used herein, the term "OX40-associated inflammatory or autoimmune disease" refers to a disease caused by OX40-mediated harmful T cell activation. The disease includes atopic dermatitis, rheumatoid arthritis, asthma, chronic obstructive pulmonary disease, inflammatory bowel disease, peritonitis, allergic rhinitis, allergic urticaria, Crohn's disease, Hirschsprung's disease, Graves' disease, Addison's disease, Guillain-Barré syndrome, Hashimoto's disease, allergic intraocular inflammatory diseases, ankylosing spondylitis, autoimmune hemolytic anemia, autoimmune hepatitis, Behcet's disease, Cogan's syndrome, contact dermatitis, Cushing's syndrome, dermatomyositis, diabetes, discoid lupus erythematosus, lupus nephritis, eosinophilic fasciitis, erythema nodosum, exfoliative dermatitis, focal or segmental glomerulosclerosis, giant cell arteritis, gout, gouty arthritis, hand eczema, Henoch-Schonlein purpura, herpes gestationis, hirsutism, idiopathic keratoscleritis, idiopathic thrombocytopenic purpura, inflammatory skin diseases, multiple sclerosis, myasthenia gravis, myositis, osteoarthritis, pancreatitis, pemphigoid gestationis, pemphigus vulgaris, periodontitis, polyarteritis nodosa, polymyalgia rheumatica, pruritus scroti, pruritus/inflammation, psoriasis, psoriatic arthritis, pulmonary histoplasmosis, relapsing polychondritis, rosacea, sarcoidosis, scleroderma, septic shock syndrome, shoulder tendonitis or bursitis, Sjogren's syndrome, Still's disease, Sweet' disease, systemic lupus erythematosus, systemic sclerosis, Takayasu's arteritis, temporal arteritis, toxic epidermal necrolysis, graft rejection, tuberculosis, ulcerative colitis, uveitis, vasculitis, Wegener's granulomatosis, Goodpasture's syndrome, Miller-Fisher syndrome, pulmonary biliary cirrhosis, paroxysmal cold hemoglobinuria, antiphospholipid syndrome, cold agglutinin disease, and IgA nephropathy.

As used herein, the inflammatory or immune disease includes, but is not limited to, immune diseases or conditions, chronic or acute inflammatory diseases, graft rejection, graft-versus-host disease (GVHD), autoimmune conditions or diseases such as rheumatoid arthritis, multiple sclerosis, diabetes (e.g., insulin dependent diabetes mellitus (IDDM, type I diabetes)), Crohn's disease (CD), inflammatory bowel disease (IBD), ulcerative colitis (UC), celiac disease, psoriasis, systemic lupus erythematosus (SLE), proliferative lupus nephritis, granulomatous myopathy, polymyositis, hyperresponsiveness/hypersensitivity (e.g., asthma and allergic asthma), and lung inflammation in other tissues and organs.

The DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody encoding sequence, a transcription termination signal, and a polyA tail. Other elements include enhancers, Kozak sequences, and donor and acceptor sites flanking the inserted sequence for RNA splicing. Efficient transcription can be achieved by early and late promoters of SV40, long terminal repeats from retroviruses such as RSV, HTLV1, and HIVI, and early promoters of cytomegalovirus, and promoters from other cells such as actin promoter can also be used. Suitable expression vectors may include pIRESlneo, pRetro-Off, pRetro-On, PLXSN, pLNCX, pcDNA3.1 (+/-), pcDNA/Zeo (+/-), pcDNA3.1/Hygro (+/-), PSVL, PMSG, pRSVcat, pSV2dhfr, pBC12MI, pCS2, etc. Commonly used mammalian cells include HEK293 cells, Cos1 cells, Cos7 cells, CV1 cells, murine L cells, CHO cells, and the like.

## DETAILED DESCRIPTION

The technical solution of the present disclosure will be further illustrated below through specific examples, which are not intended to limit the protection scope of the present disclosure. Some nonessential modifications and adjustments made by other people according to the concept of the present disclosure still fall within the protection scope of the present disclosure.

The materials, reagents, and the like used in the following examples can be commercially available unless otherwise stated.

Example 1. Preparation Method for Antibodies

The DNA sequences of the heavy chain and light chain of the antibodies were cloned into expression vectors, and then host cells were transfected with the vectors. The supernatants were collected and purified to give the antibodies, which were used in the following various examples.
1) The cells expressing antibody M-KF were CHO-BAT-KF cells (see ZL201810910890.8 for the preparation method), and the fucosylation level was 0 as tested; 2) the cells expressing antibody M were CHO-K1 cells; 3) the cells expressing antibody KHK4083 were CHO-BAT-KF cells, and the fucosylation level was 0 as tested. The amino acid sequences of antibodies M and M-KF are shown in Table 1, and the amino acid sequences of antibody KHK4083 are shown in Table 2.

Table 2. Amino acid sequences of antibody KHK4083 (US 2018/0298107 A1)

| Name | SEQ ID NO: | Amino acid sequence |
|---|---|---|
| Heavy chain | 11 | QITLKESGPTLVKPKQTLTLTCTFSGFSLSTSGMGVGWIRQPPGKA LEWLAVIYWDDHQLYSPSLKSRLTITKDTSKNQVVLTMTNMDPV DTATYYCAHRRGAFQHWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTH TCPPCPAPELLGGPSVFLFPPKPKDTLMISRTPEVTCVVVDVSHED PEVKFNWYVDGVEVHNAKTKPREEQYNSTYRVVSVLTVLHQD WLNGKEYKCKVSNKALPAPIEKTISKAKGQPREPQVYTLPPSRDE LTKNQVSLTCLVKGFYPSDIAVEWESNGQPENNYKTTPPVLDSDG SFFLYSKLTVDKSRWQQGNVFSCSVMHEALHNHYTQKSLSLSPG K |
| Light chain | 12 | EIVLTQSPGTLSLSPGERATLSCRASQSVSSSYLAWYQQKPGQAPR LLIYGASSRATGIPDRFSGSGSGTDFTLTISRLEPEDFAVYYCQQYD SSLTFGGGTKVEIKRTVAAPSVFIFPPSDEQLKSGTASVVCLLNNF YPREAKVQWKVDNALQSGNSQESVTEQDSKDSTYSLSSTLTLSK ADYEKHKVYACEVTHQGLSSPVTKSFNRGEC |

Example 2. Assay for Detecting the Blocking Effect of Antibody M-KF on OX40L Binding to OX40 on Cell Surface

In a 96-well U-shaped plate, antibody M-KF, antibody KHK4083 (US 2018/0298107 A1), and hIgG1 antibody (Sino Biological, Cat. No.: MA14OC2903) were separately diluted to an initial concentration of 300 nM with $1\times$ PBS, followed by 3-fold serial dilution with $1\times$ PBS to obtain a total of 10 dilution concentrations.

Jurkat cells overexpressing human OX40 (Jurkat-hOX40 cells) were generated by transfection with a pCMV vector carrying human OX40 cDNA. The pGL6-NFκB-luc reporter gene plasmid (purchased from Beyotime, Cat. No.: D2206) was then electrotransfected into Jurkat-hOX40 cells. After pressurized antibiotic selection, a stable monoclonal strain was obtained and designated as PG-Jurkat-hOX40-NF-κB cells.

PG-Jurkat-OX40-NF-κB cells were resuspended in $1\times$ PBS to achieve a density of $1 \times 10^7$ cells/mL. Sample wells, control wells, and blank wells were set in a 96-well V-bottom plate (Corning, Cat. No.: 3897). PG-Jurkat-OX40-NF-κB cells were plated in sample wells, control wells, and blank wells at 50 μL/well, and the cell count was $5 \times 10^5$ cells/well at this point. Antibody diluents at different concentrations were added to sample wells at 50 μL/well, and the mixture was

mixed well. 50 $\mu$L of 1$\times$ PBS was added to control wells and blank wells. The 96-well V-bottom plate was incubated in a refrigerator at 4 °C for 5 min. During the antibody incubation, an appropriate amount of ligand OX40L-mFc (Acro, Cat. No.: OXL-H5250) was diluted to 4.5 $\mu$g/mL with 1$\times$ PBS to give a diluted ligand solution. After the incubation was complete, the 96-well V-bottom plate was taken out. The diluted ligand solution was added to sample wells and control wells at 50 $\mu$L/well as soon as possible, and the mixture was mixed well by pipetting. At this point, the final concentration of ligand OX40L-mFc was 1.5 $\mu$g/mL, and 50 $\mu$L of 1$\times$ PBS was added to blank wells. The 96-well V-bottom plate was incubated in a refrigerator at 4 °C for 50 min. The plate was washed with 1$\times$ PBS, and the supernatant was removed for further incubation.

Anti-mFc APC (Bioss, Cat. No.: BJ01138707) was diluted with 1$\times$ PBS at a ratio of 1:800, and 100 $\mu$L of the diluted anti-mFc APC was added to each well except for blank wells to resuspend the cells. The plate was incubated in the dark in a refrigerator at 4 °C for 30 min.

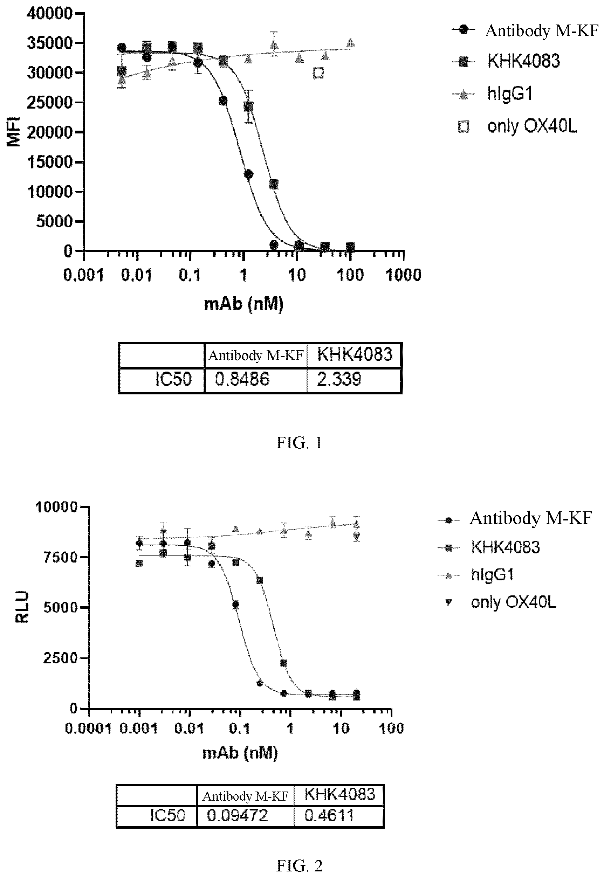

After the incubation, the cells were washed with 1$\times$ PBS and finally resuspended in 1$\times$ PBS at 200 $\mu$L/well for flow cytometry analysis. Curve fitting was performed on the final results using a four-parameter model with antibody concentration as the abscissa and MFI (mean fluorescence intensity) as the ordinate by GraphPad Prism software, and FIG. 1 was obtained.

The results indicate that antibody M-KF can significantly block the binding of OX40L to OX40 on the cell surface with an IC50 of 0.85 nM, which is 2.8 times lower than that of antibody KHK4083 of the same kind (IC50 = 2.4 nM).

Example 3. Cell Assay for Detecting Antibody M-KF Blocking of Ligand OX40L from Activating OX40

55 mL of FBS (ExCell Biology, Cat. No.: FSP500) was added to 500 mL of RPMI 1640 medium (Gibco, Cat. No.: 11875-093) to prepare an assay diluent.

Appropriate amounts of antibody M-KF, antibody KHK4083, and hIgG1 antibody were diluted to an initial concentration of 60 nM with the assay diluent, followed by 3-fold serial dilution with the assay diluent to obtain a total of 10 dilution concentrations.

PG-Jurkat-OX40-NF-$\kappa$B cells were resuspended in the assay diluent to achieve a cell density of $1 \times 10^6$ cells/mL. Sample wells, negative control wells, and blank wells were set in a 96-well white plate (Corning, Cat. No.: 3917). PG-Jurkat-OX40-NF-$\kappa$B cells were plated in sample wells and negative control wells at 50 $\mu$L/well, and the cell count was $5 \times 10^4$ cells/well at this point. Antibody dilutions at different concentrations were added to sample wells at 50 $\mu$L/well, and the mixture was mixed well. The assay diluent was added to negative control wells at 50 $\mu$L/well. 100 $\mu$L of RPMI 1640 medium was added to the surrounding blank wells, and the 96-well white plate was then incubated in a thermostatic incubator with $CO_2$ at 37 °C for 30 min. During the antibody incubation, an appropriate amount of ligand OX40L-mFc was diluted to 4.5 $\mu$g/mL with the assay diluent to give a ligand diluent. The 96-well white plate was taken out, and the ligand diluent was added at 50 $\mu$L/well as soon as possible. The mixture was mixed well by pipetting, and the final concentration of ligand OX40L-mFc was 1.5 $\mu$g/mL. The 96-well white plate was then incubated in a thermostatic incubator with $CO_2$ at 37 °C for 6 h. After the incubation, the 96-well white plate was taken out from the incubator and equilibrated at room temperature in a super clean bench for about 8 min. 70 $\mu$L of Bio-Lite™ Luciferase Assay System color-developing solution (Nanjing Vazyme Biotech Co., Ltd., Cat. No.: DD1201-03) was added to each well, and the plate was shaken for 30 s, left to stand in the dark at room temperature for 5 min, and read as soon as possible.

The relative light unit (RLU) was read using the luminescence detection module on a multimode microplate reader with PMT and Optics set to 500. The plate was shaken for 10 s before reading. The experimental data results were processed using GraphPad Prism software, and curve fitting was performed for the samples using a 4-parameter model with antibody concentration as the abscissa and relative light unit as the ordinate. A four-parameter curve was obtained, as shown in FIG. 2. It can be seen that antibody M-KF can significantly block the activation of OX40 signals by OX40L with an IC50 of 0.095 nM, which is 4.8 times lower than that of antibody KHK4083 of the same kind (IC50 = 0.46 nM).

Example 4. Assay for Mixed Lymphocyte Reaction (MLR)

1 day before the experiment, PBMCs (Leide Biosciences Co., Ltd.) from different donors (donor A and donor B) purchased on that day were transferred into a T25 cell culture flask and cultured in a thermostatic incubator with $CO_2$ at 37 °C overnight.

Dissolved mitomycin C was added to a PBMC suspension of donor A at a final concentration of 50 $\mu$g/mL. The mixture was mixed well, incubated in a thermostatic incubator with $CO_2$ at 37 °C for 30 min, and washed twice with RPMI 1640 medium. After resuspension in RPMI 1640 medium containing 10% FBS, an appropriate amount of RPMI 1640 medium containing 10% FBS was added to enable the PBMC cells of donor A to achieve a density of $1 \times 10^6$ cells/mL and the PBMC cells of donor B to achieve a density of $1.6 \times 10^6$ cells/mL. After the treatment, the PBMC cells of donor A were defined as stimulator cells, and the PBMC cells of donor B were defined as responder cells.

Appropriate amounts of antibody M-KF, antibody KHK4083, antibody M, Abatacept (Bristol-Myers Squibb Investment Co. Ltd., ABR4739), and hIgG1 antibody were diluted to an initial concentration of 200 nM with RPMI 1640 medium containing 10% FBS, followed by 10-fold serial dilution with an assay diluent. Only the highest 2 serial dilution concentrations were designed for Abatacept, and 6 serial dilution concentrations were obtained for each of the other four antibodies.

The stimulator cells and the responder cells were added to a 96-well U-shaped plate at 50 $\mu$L/well, i.e., $5 \times 10^4$ cells/well of stimulator cells and $8 \times 10^4$ cells/well of responder cells, and antibody diluents at different concentrations were added at 100 $\mu$L/well. The mixture was mixed well, and the 96-well U-shaped plate was incubated in a thermostatic incubator with $CO_2$ at 37 °C for 7 days.

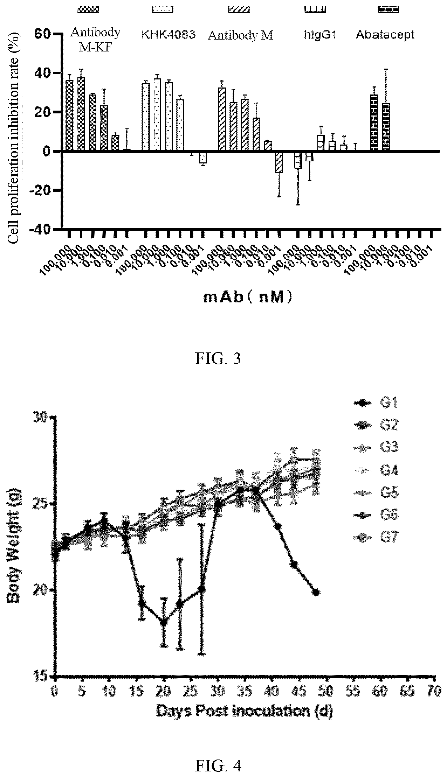

Detection of cell proliferation: CCK-8 Cell Counting Kit was added to each well at a volume equivalent to 10 % of the final well volume, and the mixture was mixed well while avoiding the generation of bubbles and protected from light. The plate was incubated in a thermostatic incubator with $CO_2$ at 37 °C for 2.5 h. The 96-well U-shaped plate was taken out from the incubator, and the condensation at the bottom of the plate was wiped off. The OD450 was measured on a microplate reader using the Endpoint type of the ABS mode. GraphPad Prism software was used to generate a column graph with antibody concentration as the abscissa and percentage of cell inhibition as the ordinate, demonstrating cell proliferation status, as shown in FIG. 3.

It can be seen that compared with the negative control hIgG1, antibody M-KF can significantly inhibit the proliferation of lymphocytes, and its inhibition efficiency is comparable to that of antibody KHK4083 of the same kind. Antibody M also has a certain inhibitory effect on lymphocyte proliferation.

Example 5. Pharmacodynamic Evaluation of Antibodies in GVHD Mouse Model

NCG mice (GemPharmatech Co., Ltd.) were divided into 7 groups based on body weight, with 10 mice per group. Reconstitution was performed by injection of $1.5 \times 10^7$ PBMCs (GemPharmatech, A19Z260104) via the tail vein. The day of reconstitution was designated as D0. The administration was performed once the day before reconstitution (designated as D-1), followed by administration on D4 after reconstitution and then once every 3 days thereafter for a total of 10 administrations. All administrations were performed by intraperitoneal injection with an administration volume of 200 $\mu$L. The specific grouping and doses are shown in Table 3.

Table 3. Grouping and administration doses

| Group | Number of animals (mice) | Treatment group | Administration dose | Route of administration |
|---|---|---|---|---|
| G1 | 10 | hIgG1 | 23 $\mu$g | i.p. |
| G2 | 10 | Antibody M-KF | 115 $\mu$g | i.p. |
| G3 | 10 | Antibody M-KF | 23 $\mu$g | i.p. |
| G4 | 10 | Antibody M-KF | 4.6 $\mu$g | i.p. |
| G5 | 10 | Antibody KHK4083 | 115 $\mu$g | i.p. |
| G6 | 10 | Antibody KHK4083 | 23 $\mu$g | i.p. |
| G7 | 10 | Antibody KHK4083 | 4.6 $\mu$g | i.p. |
| Note: At the time of initial administration, 23 $\mu$g was equivalent to about 1 mpk, 4.6 $\mu$g was about 0.2 mpk, and 115 $\mu$g was about 5 mpk. | | | | |

After the initial administration, all abnormal manifestations or symptoms observed during the experiment were recorded in the raw data. The body weight and clinical pathological assessment of the mice were observed twice a week. During the administration period, clinical scoring was also performed based on clinical observations, including evaluations of body weight, mouse posture, mobility, fur, skin, etc., and the scoring criteria are shown in Table 4.

Table 4. Clinical GVHD scoring of graft-bearing animals

| Criteria | Grade 0 (0 points) | Grade 1 (1 point) | Grade 2 (2 points) |
|---|---|---|---|
| Weight loss | <5% | 5%-10% | >10% |
| Posture | Normal | Hunched back at rest | Severely hunched, affecting mobility |
| Activity | Normal | Mild-to-moderate decreased activity | Only moves when stimulated |
| Fur | Normal | Mild-to-moderate piloerection | Severe piloerection |

(continued)

| Criteria | Grade 0 (0 points) | Grade 1 (1 point) | Grade 2 (2 points) |
|---|---|---|---|
| Skin | Normal | Scaling on paws and tail | Significant exposed skin (severe alopecia) |

Changes in the body weights of the mice during the administration period are shown in FIG. 4. According to the statistical analysis of the body weight data on D20, the body weights of groups G2, G3, G4, G5, G6, and G7 are significantly statistically different from that of the control group G1.

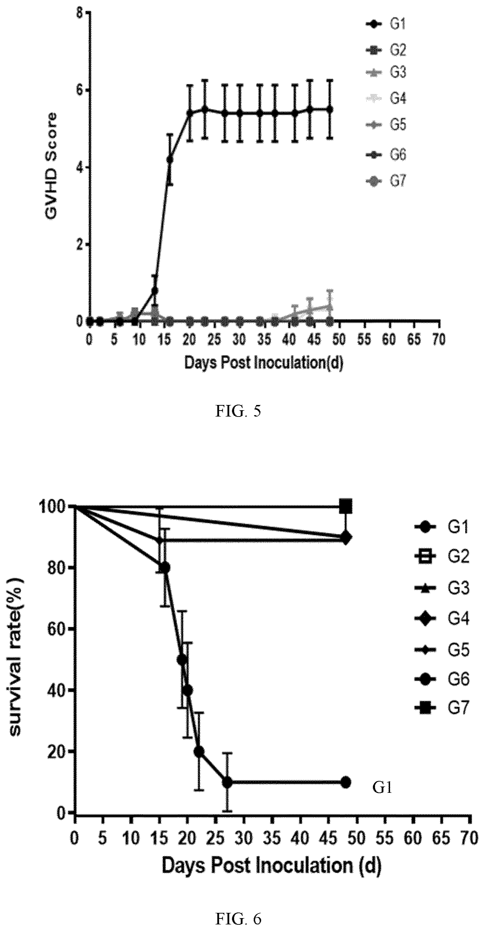

The GVHD scores of the mice in different groups throughout the administration period are shown in FIG. 5. According to the statistical analysis of the GVHD score data on D48, the GVHD score data of groups G2, G3, G4, G5, G6, and G7 are significantly statistically different from that of the control group G1.

In addition, the survival of the mice during the administration period is shown in FIG. 6. According to the statistical analysis of the survival curve data, the survival time of groups G2, G3, G4, G5, G6, and G7 is significantly prolonged as compared with that of the control group G1.

The results of the pharmacodynamic experiment indicate that under the current test system, compared with the control group hIgG1, antibody M-KF (115 $\mu$g, 23 $\mu$g, and 4.6 $\mu$g) and antibody KHK4083 (115 $\mu$g, 23 $\mu$g, and 4.6 $\mu$g) all exhibit good therapeutic effects on GVHD. On D45, 100% of GVHD mice treated with antibody M-KF survived with almost no GVHD symptoms, and the efficacy of antibody M-KF was slightly better than that of KHK4083.

Example 6. Pharmacodynamic Mechanism Study on Antibodies in GVHD Mouse Model

In Example 4, 120 $\mu$L of blood was collected from the first 8 mice in each group on D15, >50 $\mu$L of plasma was separated for CBA cytokine detection, and the remaining blood cells were used for flow cytometry. IL-2, IL-4, IL-6, IL-10, TNF, and IFN-$\gamma$ were selected for cytokine detection, and the detection range was 20-5000 pg/mL.

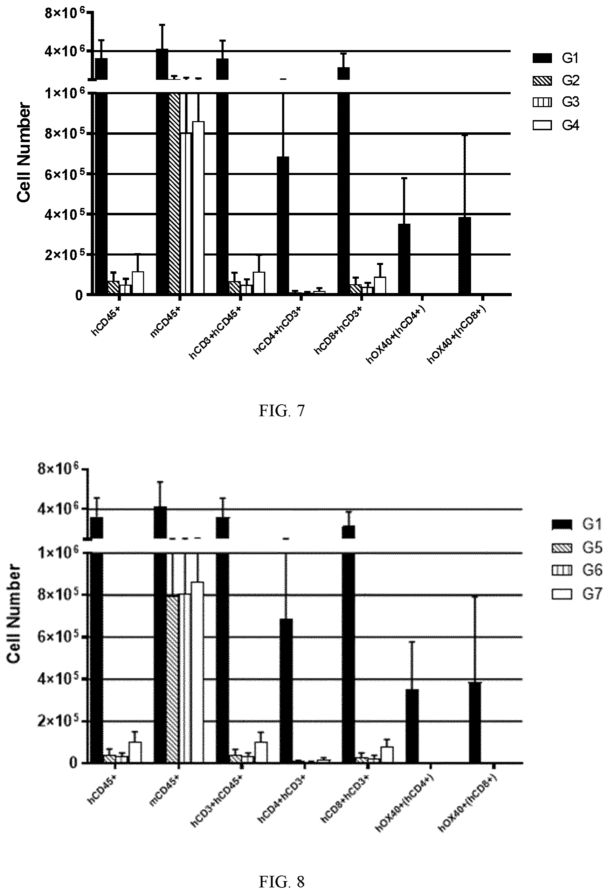

Flow cytometry was performed to detect human CD45-positive cells (hCD45+), murine CD45-positive cells (mCD45+), human CD3-positive cells (hCD3+), human CD4-positive cells (hCD4+), human CD8-positive cells (hCD8+), and human OX40-positive cells (hOX40+) in the blood cells. The results are shown in FIG. 7 and FIG. 8. The experimental results indicate that on D15, antibody M-KF significantly reduced the number of human CD3, CD4, and CD8+ T cells in the blood of GVHD mice, with the number of OX40+ T cells reduced to almost zero.

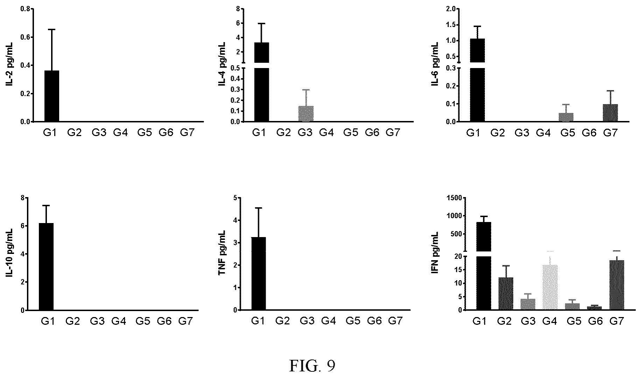

The contents of cytokines IL-2, IL-4, IL-6, IL-10, TNF, and IFN-$\gamma$ in plasma were determined using the Cytometric Bead Array (CBA) Human Th1/Th2 Cytokine Cytometric Bead Array (CBA) Kit II (BD, Cat. No.: 551809). The detection results are shown in FIG. 9. The experimental results indicate that antibody M-KF also significantly reduced the contents of human cytokines IL-2, IL-4, IL-6, IL-10, TNF, and IFN-$\gamma$ in the blood of GVHD mice.

Example 7. Phase I/II Clinical Study for Evaluating Safety and Efficacy of Antibody M-KF in Patients with Moderate-to-Severe Atopic Dermatitis

This study is a phase I/II clinical study for evaluating the safety and efficacy of antibody M-KF in patients with moderate-to-severe atopic dermatitis. Subjects of the study were adult patients with moderate-to-severe atopic dermatitis (AD) whose disease cannot be adequately controlled by previous topical medications or is not amenable to treatment with topical medications. Stage 1 was a phase I study, in which a "3 + 3" dose escalation study across 3 dose levels was carried out. Upon completion of the tolerability study, the trial proceeded to the phase II study, a randomized, double-blind, placebo-controlled, multicenter clinical trial with different doses. The primary endpoints were the proportion of patients achieving at least 75% reduction in the Eczema Area and Severity Index (EASI) at week 20 after 5 treatment cycles as compared with the baseline and the proportion of subjects achieving an Investigator's Global Assessment (IGA) score of 0 or 1 with at least a two-grade reduction as compared with the baseline. These endpoints were used to explore the efficacy and safety of antibody M-KF and to provide the optimal administration dose for the phase III protocol.

The study was divided into two stages:

**Stage 1: Phase I dose escalation study.** The "3 + 3" dose escalation rule was used to explore the safety and tolerability of antibody M-KF in adult AD patients. A total of 3 dose groups were set, i.e., a 300 mg group, a 600 mg group, and a 900 mg group, which were administered once every 4 weeks (Q4W). After the administration in cycle 1, an additional equivalent dose was given at week 2. The dose escalation study was performed according to the standard "3 + 3" rule, as shown below:

| Group | Dose | Administration interval | Description |
|---|---|---|---|
| A1 | 300 mg | Q4W | An additional dose of 300 mg was given at week 2 after the administration in cycle 1. |

(continued)

| Group | Dose | Administration interval | Description |
|---|---|---|---|
| B1 | 600 mg | Q4W | An additional dose of 600 mg was given at week 2 after the administration in cycle 1. |
| C1 | 900 mg | Q4W | An additional dose of 900 mg was given at week 2 after the administration in cycle 1. |

**The dose escalation rule was as follows:**
The dose escalation pattern of the standard "3 + 3" rule was as shown in the table below:

| Number of subjects with DLT/number of subjects in current dose group | Dose escalation/de-escalation decision |
|---|---|
| 0/3 | 3 new subjects were enrolled in the next higher dose group |
| 1/3 | 3 new subjects were enrolled in the current dose group |
| 1/3 + 0/3 (i.e., 1/6) | 3 new subjects were enrolled in the next higher dose group |
| $\geq$ 2/3 or $\geq$ 2/6 | It is indicated that this dose exceeded the MTD, and dose escalation should be stopped. If 6 subjects were enrolled in the previous dose group, the previous dose should be used as the MTD. If only 3 subjects were enrolled in the previous dose group, 3 more subjects should be enrolled in the previous dose group. After 3 more subjects were enrolled, if there were a total of $\geq$2 DLTs in the previous dose group, the MTD should be de-escalated again. |

If the MTD was identified, two dose levels, either the MTD or a dose lower than the MTD, could be selected as the doses for the phase II clinical study based on the specific situation and the investigator's discussion.

During the tolerability exploration according to the standard "3 + 3" dose escalation rule, all subjects in the previous dose group must complete a 28-day DLT observation before the enrollment for the next dose group (cohort). In the dose escalation study, the time interval for the first administration between one subject and the next subject was at least 24 h in each dose group.

**The endpoints of the phase I dose escalation study were safety indexes:** vital signs, physical examinations, adverse events, laboratory tests, electrocardiogram, cardiac color ultrasound, ECOG scores, and the like.

**Stage 2: Randomized, double-blind, placebo-controlled, multicenter phase II clinical study with different doses.** 3 dose groups were set, i.e., A2, B2, and C2, which were administered once every 4 weeks (Q4W). After the administration in cycle 1, an additional corresponding dose was given at week 2, as shown below.

| Group | Dose | Administration interval | Description |
|---|---|---|---|
| A2* | 600 mg | Q4W | An additional dose of 600 mg was given at week 2 after the administration in cycle 1. |
| B2* | 900 mg | Q4W | An additional dose of 900 mg was given at week 2 after the administration in cycle 1. |
| C2 | Placebo | Q4W | Placebo was additionally given at week 2 after the administration in cycle 1. |
| *Specific doses for groups A2 and B2 were to be determined based on the results of the phase I dose escalation study. | | | |

**Study duration:** The study consisted of a treatment period of up to 20 weeks (a total of 6 administrations in 5 administration cycles with an end of therapy (EOT) visit completed) and a subsequent follow-up period of 12 weeks. For each subject, the overall study period was roughly divided into a screening period, a treatment period (until discontinuation), and a follow-up period.

**Treatment period:** The treatment period consisted of 5 administration cycles with every 4 weeks as 1 administration cycle (Q4W). Treatment with the study drug was performed on day 1 in each cycle. The study drug was additionally administered once every 2 weeks after the administration in cycle 1. From cycle 2, a time window of $\pm 3$ days for administration was permitted, but within 72 h before each administration, the subjects were required to complete various safety assessments, such as vital signs, physical examinations, laboratory tests, and adverse events, and to complete relevant assessments of efficacy. The treatment period consisted of 5 administration cycles with an EOT visit completed 4 weeks after the last administration. The visit should include various examinations, such as vital signs, physical examinations, laboratory tests, performance status scores, adverse events, efficacy evaluation, and concomitant medication. If clinical laboratory tests (e.g., complete blood count, blood biochemistry, urinalysis, and coagulation function) have been performed within $\leq 7$ days before the EOT visit, they are not required to be performed again. AEs were to be monitored until their severity had decreased to grade $\leq 1$, or until they had returned to baseline levels, or until they had stabilized in a state where the investigator could provide a clinically reasonable explanation, or until the subject was lost to follow-up. If a subject was unable to return to the study site for the EOT visit, every effort was to be made to contact the subject by means such as telephone or e-mail to collect information on AEs and concomitant therapy. Patients who required remedial treatment for their disease during the treatment period were to be withdrawn from the trial and were to complete the EOT visit.

**Phase II efficacy evaluation indexes:** The efficacy of antibody M-KF in AD patients was evaluated by: the proportion of patients achieving at least 75% reduction in the Eczema Area and Severity Index (EASI) at week 20 as compared with the baseline; the proportion of patients achieving at least 75% reduction in the Eczema Area and Severity Index (EASI) at week 24 and week 32 as compared with the baseline; the proportion of subjects achieving an Investigator's Global Assessment (IGA) score of 0 or 1 with at least a two-grade reduction as compared with the baseline; changes in indexes such as SCORing Atopic Dermatitis (SCORAD) Criteria, AD Skin Lesion Body Surface Area (BSA), Itch Severity NRS, Sleep Disorder NRS, and Dermatology Life Quality Index (DLQI) at week 20, week 24, and week 32; the proportions of subjects achieving: EASI-50 ($\leq 50\%$ reduction in EASI score as compared with the baseline) and EASI-90 ($\geq 90\%$ reduction in EASI score as compared with the baseline); the proportion of subjects achieving $\geq 2$-point reduction in IGA score as compared with the baseline; the proportion of subjects achieving an Investigator's Global Assessment (IGA) score of 0 or 1 with at least a two-grade reduction as compared with the baseline. The relationship between the efficacy of antibody M-KF and the expression levels of AD disease markers (serum thymus and activation-regulated chemokine (TARC), total serum IgE) was explored.

Administration was performed by intravenous infusion once every 4 weeks (Q4W). The study drug was additionally administered once at week 2 after the administration in cycle 1. A total of 5 administration cycles were scheduled. Administration was performed by intravenous infusion for a recommended infusion duration of $\geq 60$ min at intervals as required by the protocol. Administration was performed by intravenous infusion for a recommended infusion duration of $\geq 60$ min at intervals as required by the protocol. If the patient had infusion-related reactions, the investigator could consider using a prophylactic administration of drugs such as diphenhydramine or acetaminophen based on previous experience and clinical practice. If no infusion-related reactions were observed, the subsequent infusion time could be adjusted to 30 min - 2 h by the investigator according to the actual clinical practice to complete the infusion.

DLT evaluation period: Applicable only to the phase I study, defined as the first treatment cycle, i.e., from the first administration to the end of day 28 after administration.

**Safety indexes involve:** vital signs, physical examinations, adverse events, laboratory tests, electrocardiogram, cardiac color ultrasound, ECOG scores, and the like.

**Immunogenicity evaluation indexes involve:** the sample positive rate and individual positive rate for the anti-drug antibody (ADA), the titer of the ADA-positive sample, and whether the ADA-positive sample will continue to be tested for a neutralizing antibody (NAb).

**Pharmacokinetic (PK) characteristics:** pharmacokinetic evaluation: the serum concentration of antibody M-KF for injection at time points specified in the protocol; pharmacokinetic parameters after single and multiple administrations, including:

single administration: $C_{max}$, $T_{max}$, $T_{1/2}$, CL, Vd, Ke, MRT, $AUC_{(0-\tau)}$, and $AUC_{(0-\infty)}$; multiple administrations: $C_{max, \, ss}$, $C_{avg, \, ss}$, $C_{min, \, ss}$, $AUC_{(0-\tau)ss}$, $AUC_{(0-\infty)ss}$, $T_{max, \, ss}$, $T_{1/2, \, ss}$, CL, Vss, Ke, MRT, accumulation index (Rac), and fluctuation index (DF).

DLT and adverse events (AEs) were evaluated according to CTCAE 5.0 criteria. Dose-limiting toxicity (DLT) is defined as an AE that occurs during the DLT observation period and is considered to be at least possibly related to the study drug, as follows:

**Grade 5 hematological toxicities of any type:**

**Hematological toxicity:**

Grade 4 hematological toxicity (note: grade 3-4 lymphopenia was not included in DLT);

Grade 4 thrombocytopenia of any duration;

Grade 3 thrombocytopenia with a bleeding tendency or requiring platelet infusion;

Grade 4 neutropenia of any duration;

Grade 3 neutropenia with infection or lasting ≥7 days or grade 3 neutropenia with fever.

**Non-hematological toxicity:**

Grade 3 or 4 non-hematological toxicity (note: (1) except for grade 3 nausea, vomiting, and rash; (2) except for grade 3 diarrhea that can be controlled within 3 days after the clinical intervention; (3) except for fatigue/weakness that lasts less than 7 days);

Grade 3 or 4 non-hematological toxicity by clinical laboratory tests that meet any one of the following conditions: (1) requiring clinical intervention; (2) leading to hospitalization; (3) lasting ≥7 days; (4) all that results in the discontinuation of treatment in the subject in cycle 1; (5) any treatment-related toxicity that results in a delay of more than 2 weeks in the administration of cycle 2;

Grade ≥2 brain lesions;

Ocular toxicity requiring systemic treatment (note: any patient with changes in visual acuity or ocular toxicity of grade 2 or higher will be evaluated by an ophthalmologist).

Example 8. Phase IB/IIA Clinical Study for Evaluating Safety and Efficacy of Antibody M-KF in Patients with Moderate-to-Severe Atopic Dermatitis

This study is a phase IB/IIA clinical study of antibody M-KF in AD patients. Subjects of the study were adult patients with moderate-to-severe atopic dermatitis (AD) whose disease cannot be adequately controlled by previous topical medications or is not amenable to treatment with topical medications. Stage 1 was a phase IB study, in which an intra-group, placebo-controlled, double-blind design was adopted, and 3 dose escalation groups were set. After the dose escalation study was completed, the trial proceeded to the phase IIA study. The phase IIA study was a randomized, double-blind, placebo-controlled, multicenter clinical trial for different dose groups and the placebo group. The primary endpoints were the proportion of patients achieving at least 75% reduction in the Eczema Area and Severity Index (EASI) at week 16 as compared with the baseline after 4 treatment cycles. These endpoints were used to explore the efficacy and safety of antibody M-KF and to provide the optimal administration dose for the phase III protocol.

The study was divided into two parts overall:

**Part 1 was a phase I dose escalation study: an intra-group, placebo-controlled, double-blind design was adopted, and 3 dose escalation groups were set to explore the safety and tolerability of antibody M-KF in adult AD patients.**

Determination of initial dose: Previous trials have been conducted to evaluate the safety and tolerability of antibody M-KF. In a study titled "A Multicenter, Open-Label, Dose Escalation Phase I Clinical Study for Evaluating Safety, Tolerability, Pharmacokinetic Characteristics, and Preliminary Clinical Efficacy of Antibody M-KF Injection in Patients with Advanced Solid Malignancies", the dose escalation involved 8 groups with doses of 0.01 mg/kg, 0.03 mg/kg, 0.1 mg/kg, 0.3 mg/kg, 1 mg/kg, 3 mg/kg, 6 mg/kg, and 10 mg/kg, respectively, and administration was performed once every 3 weeks. To date, 25 patients have been enrolled, and DLT observation of 3 patients in the 10 mg/kg group (equivalent to 600 mg for an average body weight of 60 kg) has been completed without any DLTs. Therefore, in this project, an initial dose of 300 mg has been selected.

In phase IB, a total of 3 dose groups were set, i.e., the 300 mg Q4W group (administered once every four weeks), the 600 mg Q4W group (administered on weeks 0, 4, 8, and 12), and the 600 mg Q2W group (administered on weeks 0, 2, 4, 6, 8, 10, 12, and 14). The doses and grouping are shown in the table below:

| Group | Dose | Administration interval |
| --- | --- | --- |
| A1 | 300 mg | Q4W |

(continued)

| Group | Dose | Administration interval |
|---|---|---|
| B1 | 600 mg | Q4W |
| C1 | 600 mg | Q2W |
| *Note 1: After the tolerability study for group A1 was completed, the doses for groups B1 and C1 could be appropriately adjusted based on the safety or PK data of the previous group.<br>*Note 2: After the administration of the last subject in group C1 was completed, whether to add a higher dose group may be discussed based on the existing safety, efficacy, and PK data. | | |

**Part 2 was a phase IIA study: a randomized, double-blind, placebo-controlled, multicenter clinical trial with different doses, with 3 groups:**

| Group | Dose | Administration interval | Number of cases | Description |
|---|---|---|---|---|
| A2* | 300 mg | Q4W | 30 | An additional dose of 300 mg was given at week 2 after the administration in cycle 1. |
| B2* | 600 mg | Q4W | 30 | An additional dose of 600 mg was given at week 2 after the administration in cycle 1. |
| C2 | Placebo | Q4W | 30 | Placebo was additionally given at week 2 after the administration in cycle 1. |
| *Note: The doses for groups A2 and B2 could be adjusted according to the results of the phase IB dose escalation study. | | | | |

**Study Duration:**

The study lasted for 16 weeks, and for each subject, the overall study period was roughly divided into a screening period and a treatment period.

(1) **Screening period:** -28d to -1d, i.e., after the informed consent is signed, the screening assessments need to be completed within 28 days. If clinical laboratory tests (e.g., complete blood count, coagulation profile, blood biochemistry, urinalysis, and coagulation function) have been performed within ≤7 days before the first administration, they are not required to be performed again.

(2) **Treatment period:** The treatment period in phase IB consisted of 4 administration cycles in total with every 4 weeks as 1 administration cycle (Q4W). The 300 mg Q4W group and the 600 mg Q4W group were administered at weeks 0, 4, 8, and 12, and the 600 mg Q2W group was administered at weeks 0, 2, 4, 6, 8, 10, 12, and 14, with the study drug being administered on day 1 of each cycle. The treatment period in phase IIA consisted of 4 administration cycles in total with every 4 weeks as 1 administration cycle (Q4W). The administration was performed at weeks 0, 4, 8, and 12. The treatment with the study drug was performed on day 1 of each cycle, but the study drug was additionally administered once at week 2 after the administration in cycle 1. From cycle 2, a time window of ±3 days for administration was permitted, but within 24 h before each administration, the subjects were required to complete various safety assessments, such as vital signs, physical examinations, laboratory tests, and adverse events, and to complete relevant assessments of efficacy. The treatment period consisted of 4 administration cycles with an end of therapy (EOT) visit completed 4 weeks after the last administration. The visit should include the contents of vital signs, physical examinations, laboratory tests, adverse events, evaluation of efficacy (including various scales), concomitant medication, and the like. If clinical laboratory tests (e.g., complete blood count, blood biochemistry, urinalysis, and coagulation function) have been performed within ≤7 days before the EOT visit, they are not required to be performed again. AEs were to be monitored until their severity had decreased to grade ≤1, or until they had returned to baseline levels, or until they had stabilized in a state where the investigator could provide a clinically reasonable explanation, or until the subject was lost to follow-up. If a subject was unable to return to the study site for the EOT visit, every effort was to be made to contact the subject by means such as telephone or e-mail to collect information on AEs and concomitant therapy. Patients who required remedial treatment for their disease during the treatment period were to be withdrawn from the trial and were to complete the EOT visit.

**Administration regimen:** Administration was performed by intravenous infusion for a recommended duration of ≥60 min for the first infusion at intervals as required by the protocol. If the patient had infusion-related reactions, the investigator could consider using a prophylactic administration of drugs such as diphenhydramine or dexamethasone for subsequent administration cycles based on previous experience and clinical practice. If no infusion-related reactions were observed, the subsequent infusion time could be adjusted to 30 min - 2 h by the investigator according to the actual clinical practice to complete the infusion.

**Objective of phase IB dose escalation study:**

The primary objective of the phase IB study includes: evaluating the tolerability and safety of antibody M-KF in patients with moderate-to-severe AD.

**Objective of phase IIA study:**

The primary objective of the phase IIA study is to evaluate the efficacy of antibody M-KF at two doses in treating patients with moderate-to-severe AD.

**Endpoints of phase IB dose escalation study**

**Primary endpoints of phase IB dose escalation study:**

- Tolerability: The incidence of experimental drug-related AEs graded as Grade 3 or higher in each dose group.

- Safety indexes: vital signs, physical examinations, adverse events, laboratory tests, electrocardiogram, and the like.

**Secondary endpoints of phase IB dose escalation study:**

- Clinical efficacy indexes: Changes of patients in indexes such as Eczema Area and Severity Index (EASI), Investigator's Global Assessment (IGA), AD Skin Lesion Body Surface Area (BSA), Itch Severity Numerical Rating Scale (NRS), and Dermatology Life Quality Index (DLQI) at weeks 4, 8, 12, and 16 as compared with the baseline.

- Immunogenicity evaluation indexes: anti-drug antibody (ADA)/neutralizing antibody (Nab).

- Pharmacokinetic evaluation: the serum concentration of antibody M-KF for injection at time points specified in the protocol; pharmacokinetic parameters after single and multiple administrations, including:

- Single administration: $C_{max}$, $T_{max}$, $T_{1/2}$, CL, Vd, Ke, MRT, $AUC_{(0-\tau)}$, and $AUC_{(0-\infty)}$;

- Multiple administrations: $C_{max,\ ss}$, $C_{avg,\ ss}$, $C_{min,\ ss}$, $AUC_{(0-\tau)ss}$, $AUC_{(0-\infty)ss}$, $T_{max,\ ss}$, $T_{1/2,\ ss}$, CL, Vss, Ke, MRT, accumulation index (Rac), and fluctuation index (DF).

**Endpoints of phase IIA study:**

**Primary endpoint:**

Clinical efficacy index: the proportion of subjects achieving at least 75% reduction in the Eczema Area and Severity Index (EASI-75) at week 16 as compared with the baseline.

**Secondary endpoints:**

Safety indexes: vital signs, physical examinations, adverse events, laboratory tests, electrocardiogram, and the like.

Efficacy indexes:

- the proportion of subjects achieving at least 75% reduction in the Eczema Area and Severity Index (EASI) at weeks 2, 4, 8, and 12 as compared with the baseline;

- changes in indexes such as AD Skin Lesion Body Surface Area (BSA), Itch Severity NRS, and Dermatology Life Quality Index (DLQI) at weeks 2, 4, 8, 12, and 16 as compared with the baseline;

- the proportions of subjects achieving EASI-50 (≥50% reduction in EASI score as compared with the baseline) and EASI-90 (≥90% reduction in EASI score as compared with the baseline) in the Eczema Area and Severity Index at weeks 2, 4, 8, and 12;

- the proportion of subjects achieving a reduction of ≥2 points in Investigator's Global Assessment score at weeks 2, 4, 8, and 12 as compared with the baseline.

- Immunogenicity evaluation indexes: anti-drug antibody (ADA)/neutralizing antibody (Nab).

- Biomarkers: disease-related indexes (thymus and activation-regulated chemokine [TARC], total serum IgE, and eosinophils) in serum were detected.

**Claims**

1. A method for treating an inflammatory or immune disease, comprising: administering to a patient in need thereof an effective amount of an anti-OX40 antibody or an antigen-binding fragment, wherein the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

2. Use of an anti-OX40 antibody or an antigen-binding fragment in treating an inflammatory or immune disease, wherein the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

3. Use of an anti-OX40 antibody or an antigen-binding fragment in preparing a medicament for treating an inflammatory or immune disease, wherein the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

4. The method or use according to any one of claims 1-3, wherein the anti-OX40 antibody or the antigen-binding fragment comprises a heavy chain variable region set forth in SEQ ID NO: 7, or a sequence having at least 80% identity to the sequence set forth in SEQ ID NO: 7, or an amino acid sequence having one or more conservative amino acid substitutions as compared to the sequence set forth in SEQ ID NO: 7; and/or the anti-OX40 antibody or the antigen-binding fragment comprises a light chain variable region set forth in SEQ ID NO: 8, or a sequence having at least 80% identity to the sequence set forth in SEQ ID NO: 8, or an amino acid sequence having one or more conservative amino acid substitutions as compared to the sequence set forth in SEQ ID NO: 8.

5. The method or use according to any one of claims 1-4, wherein a heavy chain of the anti-OX40 antibody comprises a sequence set forth in SEQ ID NO: 9, or a sequence having at least 80% identity to the sequence set forth in SEQ ID NO: 9, or an amino acid sequence having one or more conservative amino acid substitutions as compared to the sequence set forth in SEQ ID NO: 9; and/or a light chain of the anti-OX40 antibody comprises a sequence set forth in SEQ ID NO: 10, or a sequence having at least 80% identity to the sequence set forth in SEQ ID NO: 10, or an amino acid sequence having one or more conservative amino acid substitutions as compared to the sequence set forth in SEQ ID NO: 10.

6. The method or use according to any one of claims 1-5, wherein the anti-OX40 antibody has a fucosylation level of 0-10%;
or the anti-OX40 antibody is expressed by a cell in which an $\alpha$-(1,6)-fucosyltransferase gene is knocked out.

7.  The method or use according to any one of claims 1-6, wherein the inflammatory or immune disease is selected from graft-versus-host disease, inflammatory diseases, and autoimmune diseases.

8.  The method or use according to any one of claims 1-7, wherein the inflammatory or immune disease is an OX40-associated inflammatory or autoimmune disease.

9.  The method or use according to any one of claims 1-8, wherein the inflammatory or immune disease is atopic dermatitis.

10. The method or use according to any one of claims 1-9, wherein a dose of the anti-OX40 antibody for each administration is 0.6 mg to 1500 mg.

11. The method or use according to any one of claims 1-9, wherein a dose of the anti-OX40 antibody for each administration is 0.01 mg/kg-25 mg/kg.

12. A pharmaceutical composition for use in treating an inflammatory or immune disease, comprising an anti-OX40 antibody or an antigen-binding fragment, wherein the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

13. A kit, comprising an anti-OX40 antibody or an antigen-binding fragment and an instruction for directing administration of the anti-OX40 antibody or the antigen-binding fragment to a patient in need thereof, wherein
the anti-OX40 antibody or the antigen-binding fragment comprises HCDR1 set forth in SEQ ID NO: 1, HCDR2 set forth in SEQ ID NO: 2, HCDR3 set forth in SEQ ID NO: 3, LCDR1 set forth in SEQ ID NO: 4, LCDR2 set forth in SEQ ID NO: 5, and LCDR3 set forth in SEQ ID NO: 6.

FIG. 1

FIG. 2

FIG. 3

FIG. 4

FIG. 5

FIG. 6

FIG. 7

FIG. 8

FIG. 9

## INTERNATIONAL SEARCH REPORT

| International application No. |
| --- |
| **PCT/CN2024/082273** |

| A. | CLASSIFICATION OF SUBJECT MATTER |
| --- | --- |

A61K39/395(2006.01)i;  C07K16/28(2006.01)i;  C12N15/13(2006.01)i;  A61K47/68(2017.01)i

According to International Patent Classification (IPC) or to both national classification and IPC

| B. | FIELDS SEARCHED |
| --- | --- |

Minimum documentation searched (classification system followed by classification symbols)

IPC:  A61K;  C07K;  C12N

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, 中国专利生物序列检索系统, China Patent Biological Sequence Search System, Genbank, EMBL, STN, PubMed, OX40, TNFRSF4, ACT35, CD134, 抗体, 抗原结合片段, 炎症, 免疫性疾病, 特应性皮炎, antibody, antigen binding fragments, inflammation, immunological diseases, atopic dermatitis, 相关序列, related sequences

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2022105839 A1 (BIO THERA SOLUTIONS, LTD.) 27 May 2022 (2022-05-27) claims 1-5 and 11, and description, paragraph 49 | 12-13 |
| Y | WO 2022105839 A1 (BIO THERA SOLUTIONS, LTD.) 27 May 2022 (2022-05-27) claims 1-5 and 11, and description, paragraph 49 | 1-11 |
| Y | CN 111763258 A (BEIJING BIOCYTOGEN CO., LTD.) 13 October 2020 (2020-10-13) claim 1, and description, paragraphs 45-50 | 1-11 |
| A | CN 114515335 A (BIO-THERA SOLUTIONS, LTD.) 20 May 2022 (2022-05-20) entire document | 1-13 |
| A | CN 115803343 A (BIO-THERA SOLUTIONS, LTD.) 14 March 2023 (2023-03-14) entire document | 1-13 |
| A | CN 114106173 A (SHANGHAI TAIJIN BIOTECHNOLOGY CO., LTD. et al.) 01 March 2022 (2022-03-01) entire document | 1-13 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **16 May 2024** | **22 May 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/082273** |

### C. DOCUMENTS CONSIDERED TO BE RELEVANT

| Category* | Citation of document, with indication, where appropriate, of the relevant passages | Relevant to claim No. |
| --- | --- | --- |
| A | CN 115776898 A (BIO-THERA SOLUTIONS, LTD.) 10 March 2023 (2023-03-10)<br>    entire document | 1-13 |
| A | Kuang, Z.H. et al. "Development and Characterization of a Novel Anti-OX40 Antibody for Potent Immune Activation"<br>*Cancer Immunology*, Vol. 69, No. (6), 20 February 2020 (2020-02-20),<br>    pp. 939-950 | 1-13 |

Form PCT/ISA/210 (second sheet) (July 2022)

**INTERNATIONAL SEARCH REPORT**

International application No.

**PCT/CN2024/082273**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/082273**

**Box No. II        Observations where certain claims were found unsearchable (Continuation of item 2 of first sheet)**

This international search report has not been established in respect of certain claims under Article 17(2)(a) for the following reasons:

1. ☑  Claims Nos.: **1-2, 4-11**
   because they relate to subject matter not required to be searched by this Authority, namely:

   Claims 1-2 and 4-11 relate to a method for treating a disease, which falls within subject matter for which a search is not performed (PCT Rule 39.1(iv)). In the present report, a search is performed on the basis of "a pharmaceutical use of the related substance".

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/082273**

| Patent document cited in search report | | | Publication date (day/month/year) | Patent family member(s) | | | Publication date (day/month/year) |
|---|---|---|---|---|---|---|---|
| WO | 2022105839 | A1 | 27 May 2022 | US | 2023416386 | A1 | 28 December 2023 |
| | | | | EP | 4248995 | A1 | 27 September 2023 |
| CN | 111763258 | A | 13 October 2020 | | None | | |
| CN | 114515335 | A | 20 May 2022 | | None | | |
| CN | 115803343 | A | 14 March 2023 | WO | 2021238932 | A1 | 02 December 2021 |
| CN | 114106173 | A | 01 March 2022 | | None | | |
| CN | 115776898 | A | 10 March 2023 | WO | 2022007807 | A1 | 13 January 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021190431 A
- US 20180298107 A1
- DD 120103

**Non-patent literature cited in the description**

- **KABAT et al.** Sequence of Proteins of Immunological Interest. U.S. Dept. of Health and Human Services, 1991
- **HANIFIN et al.** *Exp. Dermatol.*, 2001, vol. 10, 11-18